# EUROPÄISCHE PATENTSCHRIFT

(11) **EP 1 182 977 B1**
(45) Veröffentlichungstag und Bekanntmachung des Hinweises auf die Patenterteilung: **24.04.2013**
(21) Anmeldenummer: 00941921.9
(22) Anmeldetag: 20.05.2000
(51) Int. Cl.: A61B 17/70

(54) **REPOSITIONSVORRICHTUNG FÜR DIE WIRBELSÄULE**
REPOSITIONING DEVICE FOR THE VERTEBRAL COLUMN
DISPOSITIF DE REPOSITIONNEMENT DE LA COLONNE VERTEBRALE

(30) Priorität: 07.06.1999 DE 19925708; 13.12.1999 DE 19960124
(43) Veröffentlichungstag der Anmeldung: 06.03.2002
(73) Patentinhaber: Ulrich GmbH & Co. KG, 89081 Ulm (DE)
(72) Erfinder: KLUGER, Patrick, 89155 Erbach (DE)
(74) Vertreter: Hentrich, Swen
(86) Internationale Anmeldenummer: PCT/DE2000/001654
(87) Internationale Veröffentlichungsnummer: WO 2000/074580

(56) Entgegenhaltungen:
- WO-A-88/07357
- DE-A- 4 202 748
- US-A- 5 382 248

## Beschreibung

Die Erfindung betrifft eine Repositionsvorrichtung für die Wirbelsäule mit einem Längsholm, an welchem Arme angeordnet sind, wobei sich jeder Arm mit jeweils einem Implantat verbinden läßt, das in einem Wirbelkörper eingesetzt ist, wobei am Längsholm mehr als zwei Arme derart angeordnet sind, dass die Arme Kraft in Holmlängsrichtung quer zur Armlängsrichtung aufnehmen können, welche eine Auslenkung der Arme in Richtung Längsholm verursachen würden, wobei wenigstens zwei Arme in Längsrichtung des Längsholms am Längsholm verschiebbar sind, und wobei die Repositionseinrichtung eine Aufnahmevorrichtung zur Anbringung am Längsholm aufweist, an welche sich ein Armteilstück anschließt und welche bei Abnahme des Armes am Längsholm verbleibt.

### Stand der Technik

In US 5,382,248 ist eine Vorrichtung zur Stabilisierung und Immobilisierung der Wirbelsäule offenbart, die mehrere Fixierungseinheiten aufweist. Diese Vorrichtung eignet sich zumindest teilweise zur Repositionierung von Wirbelkörpern. Bei der Vorrichtung enthält jede Fixierungseinheit einen länglichen Stab mit einer Längsnut über die obere und die untere Oberfläche. Arretierungsteile sind entlang den Stäben auf vorbestimmte Positionen verschiebbar oder drehbar beweglich, die Positionen zur Stabilisierung der Wirbelsäule entsprechen. Die Pflöcke weisen an ihrem distalen Ende zur Befestigung in der Wirbelsäule ein Gewinde auf. Das proximale Ende der Pflöcke geht durch die Längsnut in dem Stab und eine vertikale Öffnung in dem Arretierungsteil.

Eine weitere Repositionsvorrichtung ist aus der deutschen Patentschrift DE 34 14 374 C2 bekannt geworden. Die hierin beschriebene Repositionsvorrichtung dient dazu, zwischen zwei in Wirbelkörpern eingebrachten Verankerungsschrauben, die an abnehmbaren Verlängerungsstäben gefaßt werden, mit zwei an einem Längsholm angeordneten Armen eine mechanische Verbindung außerhalb der Wunde herzustellen. Durch Verschieben eines Armes kann zwischen den beiden Wirbeln zur Ausrichtung von diesen eine Zugdruckkraft ausgeübt werden.

Um mehrere Wirbel einer Wirbelsäule, die z. B. eine mehrere Wirbelkörper umfassende Lateralskoliose aufweist, gleichzeitig reponieren zu können, wird in der DE 42 02 748 A1 vorgeschlagen, ein Repositionsinstrument mit einem Längsholm sowie einem festen und einem in Längsrichtung verstellbaren Arm mit weiteren Zuginstrumenten zu versehen. Diese sind in der Lage, jeweils zwischen dem Längsholm und einem weiteren zu reponierenden Wirbelkörper mittels eines Manipulationshebels, der an einer in diesen Wirbelkörper eingesetzten Verankerungsschraube befestigt ist, eine Zugspannung aufzubauen.

Zur Reposition einer sagittalen Verkrümmung, z. B. einer Lordosefehlhaltung, können derartige Repositionsvorrichtungen nur beschränkt eingesetzt werden. Möglich ist beispielsweise ein Zusammenziehen oder Aufspreizen von zwei in Wirbelkörpern angebrachten Manipulationshebel, welche senkrecht zum jeweiligen Wirbelkörper herausstehen. Eine Korrektur einer Fehlhaltung, die sich über mehr als zwei Wirbelkörper erstreckt, ist daher aufwendig und nur abschnittsweise möglich.

### Aufgabe und Vorteile der Erfindung

Der Erfindung liegt die Aufgabe zugrunde, eine Repositionsvorrichtung der einleitend bezeichneten Art bereitzustellen, die insbesondere eine verbesserte Reponierung von mehreren Wirbelkörpern bei einer sagittalen Verkrümmung ermöglicht.

Diese Aufgabe wird ausgehend von der oben bezeichneten Repositionsvorrichtung durch den Merkmalsbestand des unabhängigen Patentanspruchs 1 gelöst. Weitere vorteilhafte Ausführungsformen sind durch die abhängigen Ansprüche 2 bis 10 gegeben. Es sind am Längsholm mehr als zwei Arme derart angeordnet, dass sie Kraft in Holmlängsrichtung quer zur Armlängsrichtung aufnehmen können, welche eine Auslenkung der Arme in Richtung Längsholm um eine Achse quer zur Holmlängsrichtung verursachen würden, wobei wenigstens zwei Arme am Längsholm in seiner Längsrichtung getrennt voneinander verschiebbar sind. Die verschiebbaren Arme sind vorzugsweise im Wesentlichen spielfrei am Längsholm geführt. Damit sind sie in der Lage Kräfte in Holmlängsrichtung ohne Auslenkung in Holmrichtung um eine Achse quer zur Holmlängsrichtung aufnehmen zu können, D.h. sobald die Arme sich an einer gewünschten Position am Längsholm befinden, ist die Repositionsvorrichtung in Holmlängsrichtung betrachtet im Wesentlichen starr. Auf diese Weise können mehr als zwei Wirbelkörper, auch unmittelbar nebeneinander liegende Wirbelkörper, bei einer sagittalen Verkrümmung zueinander ausgerichtet werden, indem sich die Wirbelkörper über die fixierte Repositionsvorrichtung aneinander abstützen. Dazu sind beispielsweise an Verankerungsschrauben, die in den Wirbelkörpern sitzen, Manipulationshebel angeordnet, die normalerweise bezüglich der Rückenfläche senkrecht von den Wirbelkörpern abstehen und mit den Armen der Repositionsvorrichtung verbunden sind. Die Position von mehr als zwei Wirbelkörpern, die miteinander in Wechselwirkung stehen, läßt sich hierdurch mit einem einzigen Repositionsvorgang durch Zusammenziehen oder Auseinanderspreizen der einzelnen mit den Armen verbundenen Hebel korrigieren. An den derart ausgerichteten Wirbelkörpern können dann über die Verankerungsschrauben Implantatbestandteile positionsexakt angebracht werden, die sich über mehr als zwei Wirbelkörper erstrecken. Als Längsholm sei beispielhaft eine Zahnstange oder eine Spindel genannt. An einem derart ausgestalteten Längsholm lassen sich die verschiebbaren Arme in Holmrichtung durch einfache Mittel genau bewegen, beispielsweise im Fall einer Spindel durch eine Rändelschraube, die von der Spindel durchdrungen wird.

In einer außerdem vorteilhaften Ausgestaltung der Erfindung sind am Längsholm mehr als zwei Arme derart angeordnet, daß sie Kräfte aufnehmen können, welche eine Verdrehung der Arme um die Längsachse des Holms bewirken würden. Durch diese Vorgehensweise kann die Repositionsvorrichtung auf die Wirbelkörper auch Kräfte aufbringen, die senkrecht zur Rückenoberfläche gerichtet sind. Hierdurch erhält man einen zusätzlichen Korrekturparameter bei z. B. der Korrektur einer Wirbelsäule mit sagittaler Verkrümmung.

Um eine hohe Flexibilität der Repositionsvorrichtung zu erzielen, wird im Weiteren vorgeschlagen, daß wenigstens ein Arm bezüglich einer Verdrehung um die Längsachse des Längsholms blockierbar ist. Beispielsweise ist im Fall einer Spindel als Längsholm, an welcher sich ein oder mehrere Arme um die Spindellängsachse zunächst verdrehen lassen, ein Feststellelement, wie beispielsweise eine Madenschraube, an den verdrehbaren Armen vorgesehen, um die Drehbewegung zu blockieren.

In einer ebenfalls günstigen Ausführungsform sind die Arme bereits verdrehsicher am Längsholm angeordnet. Beispielhaft sei die bereits oben erwähnte flache Zahnstange als Längsholm genannt, auf welcher die Arme aufgeschoben werden können.

In einer weiteren vorteilhaften Ausgestaltung der Erfindung ist wenigstens ein Arm ganz oder teilweise abnehmbar. Dies ist insbesondere im Hinblick auf die Anbringung der Repositionsvorrichtung an zum Beispiel Manipulationshebeln von bereits eingesetzten Verankerungsschrauben von Vorteil. Denn hierdurch kann die Repositionsvorrichtung mit zunächst nur zwei am Längsholm angebrachten Armen ohne große Justierprobleme an Manipulationshebeln von Implantaten angebracht werden. Anschließend können weitere Arme zunächst an der jeweiligen Implantatstelle angebracht und nach einer problemlosen Ausrichtung mit dem Längsholm verbunden werden. In diesem Zusammenhang ist es überdies günstig, wenn wenigstens ein Arm eine Aufnahmeeinrichtung zur Anbringung am Längsholm aufweist, die bei der Abnahme des Arms an diesem verbleibt. Durch diese Maßnahme läßt sich die nachträgliche Verbindung von Arm und Holm, nachdem bereits zwei oder mehr Arme mit Implantaten in Verbindung stehen, zeitsparend und durch einfache Handgriffe bewerkstelligen.

Um eine sichere Befestigung der Arme am Längsholm zu gewährleisten, wird im Weiteren vorgeschlagen, daß die Aufnahmeeinrichtung vom Längsholm durchdrungen wird.

Um unterschiedliche Abstände des Längsholms zu einem Implantat bzw. zu dessen Manipulationshebel ausgleichen zu können, wird außerdem vorgeschlagen, daß wenigstens ein Arm eine oder mehrere Gelenke für eine Abwinkelbarkeit umfaßt, wobei sich die Gelenke blockieren lassen. Vorzugsweise laufen die Gelenkachsen parallel zum Holm. Durch diese Maßnahme erhält man insbesondere bei mehreren Gelenken abwinkelbare Arme, die unabhängig von der Gelenkstellung nicht mehr oder weniger in Längsrichtung des Längsholmes ausladen. Damit behindern sich die Arme am Längsholm in keiner Arbeitsstellung gegenseitig, selbst wenn sie dicht nebeneinander positioniert sind, um beispielsweise mit drei Armen drei unmittelbar nebeneinander liegende Winkelkörper erreichen zu können.

In einer besonders vorteilhaften Ausgestaltung der Erfindung sind die Arme dergestalt, daß sie sich bezüglich des Längsholms dreifach abwinkeln und anschließend blockieren lassen. Durch diese Vorgehensweise wird einerseits erreicht, daß die Repositionsvorrichtung beliebig außerhalb einer Wunde plaziert werden kann, zum Beispiel muß sie in Seitenlage des Patienten anders als in Bauchlage angeordnet werden. Andererseits gewährleistet die dreifache Abwinkelbarkeit der Arme, daß die Angriffspunkte des Arms am Holm und entlang eines Manipulationshebels bei der Anpassung an unterschiedliche Abstände unverändert bleiben können. Das heißt, die Höhenposition eines Aufnahmekörpers in Bezug auf einen Manipulationshebel kann unabhängig von der einzustellenden Distanz zum Längsholm gewählt werden.

Schließlich ist es vorteilhaft, wenn wenigstens ein Arm verlängerbar, insbesondere teleskopierbar ist. Auf diese Weise wird eine zusätzliche Möglichkeit zur Anpassung der Vorrichtung an unterschiedliche Abstände vom Längsholm zu den Implantaten geschaffen.

### Zeichnungen

Mehrere Ausführungsbeispiele der Erfindung sind in den Zeichnungen dargestellt und unter Angabe weiterer Vorteile und Einzelheiten näher erläutert.

Im Einzelnen zeigt
- Fig. 1: eine Draufsicht auf eine Repositionsvorrichtung mit einem spindelförmigen Längsholm und drei daran angebrachten, verschiebbaren Armen,
- Fig. 2: einen abgenommenen Armabschnitt aus der Repositionsvorrichtung gemäß Fig. 1 in einer Seitenansicht an einem gestrichelt dargestellten Manipulationshebel für ein Implantat angebracht,
- Fig. 3: eine teilweise geschnittene Seitenansicht eines weiteren Armes für eine Repositionsvorrichtung mit einem spindelförmigen Längsholm,

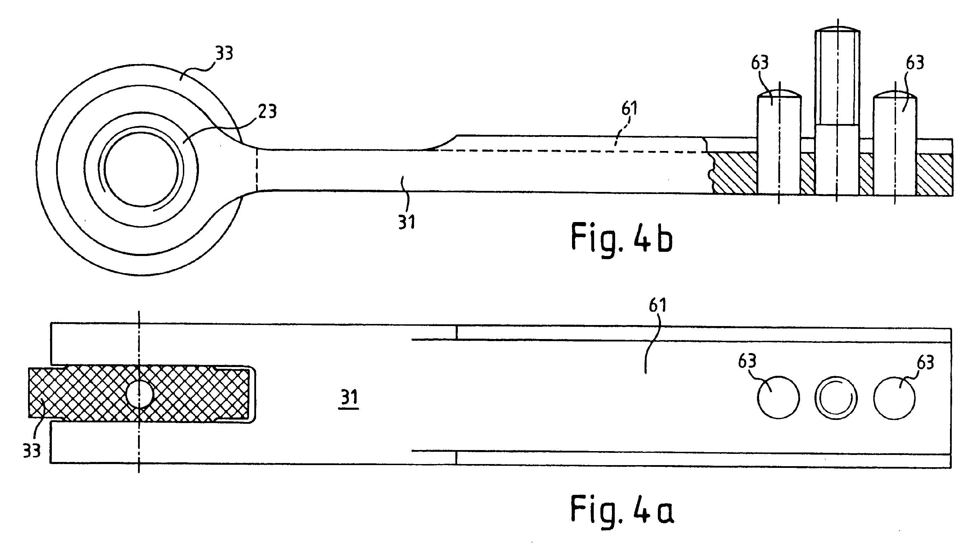

- Fig. 4a und b: eine Aufnahmeeinrichtung eines Armes gemäß Fig. 3 in einer teilweise geschnittenen Seitenansicht und einer Draufsicht,
- Fig. 5: eine vergrößerte Darstellung eines mittleren Armteilstücks mit weiteren daran angelenkten Armteilstücken aufschnittsweise und in einer teilweise geschnittenen Seitenansicht abgebildet,
- Fig. 6: einen Aufnahmekörper eines Arms gemäß Fig. 3 für einen Manipulationshebel eines Implantats in einer teilweise geschnittenen Seitenansicht und
- Fig. 7a und b: der in Fig. 6 dargestellte Aufnahmekörper für einen Manipulationshebel isoliert in einer geschnittenen Seitenansicht und einer Frontansicht dargestellt.

### Beschreibung der Ausführungsbeispiele:

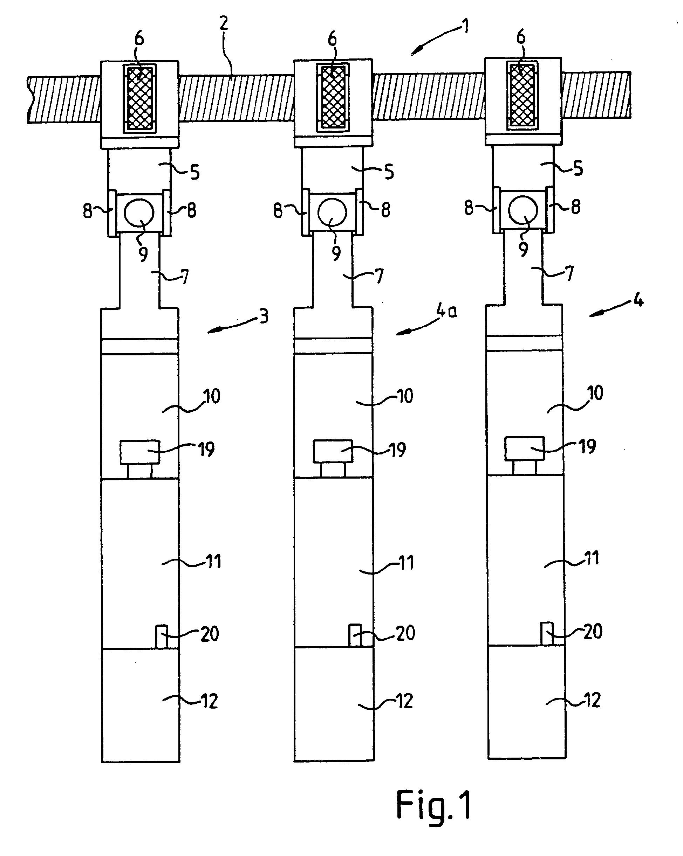

In Fig. 1 ist eine Repositionsvorrichtung 1 ausschnittsweise dargestellt. Sie umfaßt einen Längsholm 2 in Form einer Schraubenspindel, an welcher drei Arme 3, 4, 4a verschiebbar angeordnet sind.

Jeder Arm besitzt eine Aufnahmeeinrichtung 5, die von der Spindel 2 durchdrungen wird und eine Rändelmutter 6 zur Verschiebung des Armes in Längsrichtung der Spindel 2. Die Aufnahmeeinrichtung 5 wirkt mit dem Längsholm 2 derart zusammen, daß die Arme 3, 4, 4a Kräfte quer zur Armlängsrichtung, d. h. in Holmlängsrichtung, aufnehmen können.

Bei der Ausgestaltung mit Spindel 2 läßt sich jede Aufnahmeeinrichtung 5 zunächst um die Längsachse der Spindel 2 verdrehen. Als ein weiteres wesentliches Merkmal der Erfindung kann jedoch jede Aufnahmeeinrichtung 5 gegen eine derartige Verdrehung durch ein nicht gezeigtes Feststellelement, z. B. eine Madenschraube, blockiert werden.

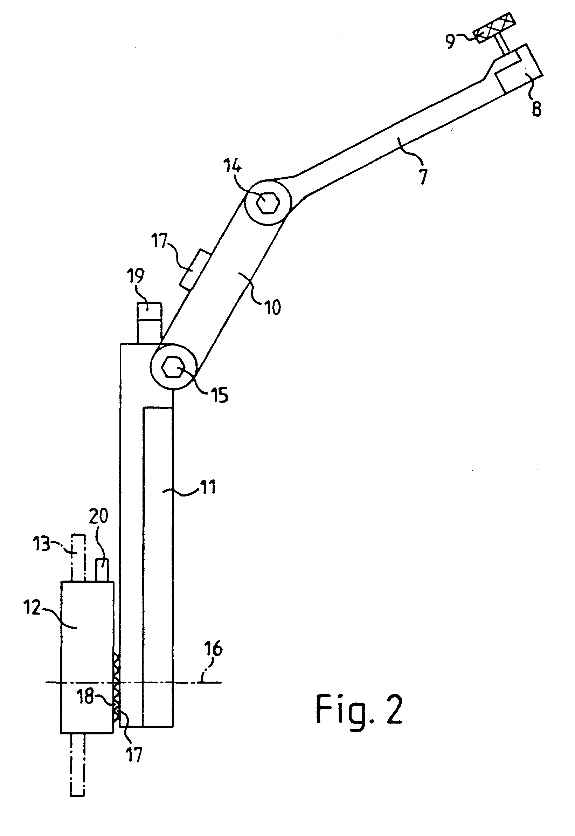

Die Arme 3, 4, 4a sind teilbar ausgeführt, das heißt, der an die Aufnahmeeinrichtung 5 anschließende Armabschnitt kann abgenommen werden. Ein Armabschnitt im abgenommenen Zustand ist in Fig. 2. dargestellt.

Um eine sichere Verbindung der Aufnahmeeinrichtung 5 mit dem restlichen Arm zu gewährleisten, kann wie in Fig. 1 schematisch und beispielhaft dargestellt, an der Aufnahmeeinrichtung 5 und am anschließenden Armteilstück 7 eine jeweils seitlich angeordnete Längsführung 8 ausgebildet sein, wodurch sich die Teile ineinander schieben lassen. Anschließend kann die Fixierung durch Anziehen einer Befestigungsschraube 9 (siehe insbesondere Fig. 2) erfolgen. Jeder Arm 3, 4, 4a weist weitere Armteilstücke 10, 11 sowie einen Aufnahmekörper 12 für einen Manipulationshebel 13 eines in einen Wirbelkörper eingesetzten Implantats auf. Die Armteilstücke 10, 11 sind jeweils über ein Gelenk 14, 15 miteinander verbunden, dessen Gelenkachse senkrecht zur Armlängsachse bzw. parallel zum Längsholm 2 im aufgesteckten Zustand des Armes verläuft. Die Gelenke 14, 15 lassen sich über ein Schraubenelement 17, welches im Armteilstück 10 angeordnet ist, lösen und blockieren. Damit läßt sich jede gewünschte Winkelstellung zwischen den Armteilstücken 7, 10 und 11 einstellen und fixieren.

Darüber hinaus kann der Aufnahmekörper 12 um eine Achse 16, die senkrecht zum Armteilstück 11 steht, gedreht werden. Zur Fixierung des Aufnahmekörpers 12 greifen am Armteilstück 11 und am Aufnahmekörper 12 angebrachte korrespondierende Rastflächen 17, 18 durch Betätigung einer im Armteilstück 11 angeordneten Spindel 19 ineinander. Zum Lösen des Aufnahmekörpers 12 wird die Spindel 19 in eine zur fixierten Stellung entgegengesetzte Drehrichtung verdreht.

Der Manipulationshebel 13 kann in den Aufnahmekörper 12 durch Drücken einer Taste 20 eingeschoben werden. Durch Freigabe der Taste 20 wird der Manipulationshebel 13 festgelegt.

Die Anordnung einer vollständigen Repositionseinrichtung 1 in Bezug auf die Wirbelkörper einer Wirbelsäule kann, wie in der oben zitierten Offenlegungsschrift DE 42 02 748 A1 in Fig. 11 dargestellt, erfolgen, wobei hier jedoch eine Lateralskoliose reponiert wird, wogegen die erfindungsgemäße Repositionsvorrichtung insbesondere zur Reposition einer Lordosefehlhaltung eingesetzt werden kann. In diesem Zusammenhang kommen dann drei oder mehr als drei Arme am Längsholm 2 zum Einsatz, die Kräfte quer zur Armlängsrichtung aufnehmen können.

Die Dreiteilung der Arme über die Gelenke 14, 15 und eine zusätzliche Verdrehmöglichkeit am Längsholm 2 erlaubt eine Einstellung des Abstandes des Aufnahmekörpers 12 zum Längsholm 2, ohne daß eine Änderung der Höhenposition des Aufnahmekörpers 12 in Bezug auf die Längsachse eines Manipulatorhebels 13 erforderlich ist. Überdies kann durch die Teilung der Längsholm 2 mit angegliederten Armteilstücken in jeder gewünschten Position außerhalb einer Wunde am Patient abgelegt werden, wodurch eine Behinderung der operierenden Person durch die Repositionsvorrichtung 1 in eleganter Weise vermieden werden kann. Durch das normalerweise zu einem Manipulatorhebel 13 parallel angeordnete, sich senkrecht von der Rückenfläche erstreckende Armteilstück 11 wird überdies sichergestellt, daß die Repositionsvorrichtung 1 nicht zu dicht an der Wunde positioniert ist.

Vorzugsweise wird für die Anbringung der Repositionsvorrichtung 1 zunächst mit zwei aufgesetzten Armen begonnen, wobei bereits eine notwendige Anzahl von weiteren Aufnahmeeinrichtungen 5 am Längsholm 2 vorpositioniert werden können. Zur weiteren Positionierung der Arme werden dann diese zunächst an den Manipulatorhebeln 13 der Implantate angebracht und bei gelösten Gelenken 14, 15 mit der jeweiligen Aufnahmeeinrichtung 5 verbunden.

Beispielsweise lassen sich auch zunächst alle abgenommenen Arme an den Implantaten anbringen, wonach sukzessive eine Verbindung der Armteilstücke 7 mit vorpositionierten Aufnahmeeinrichtungen 5 am Längsholm 2 hergestellt wird.

Nach der Anbringung der einzelnen Arme lassen sich dann deren Gelenke 14, 15 blockieren. Nachdem die Arme in gewünschter Weise angeordnet sind, läßt sich die Reposition der Wirbelkörper durch Verschieben der Arme auf dem Längsholm 2 bzw. weiteres Lösen und Blockieren der Gelenke 14, 15 herbeiführen.

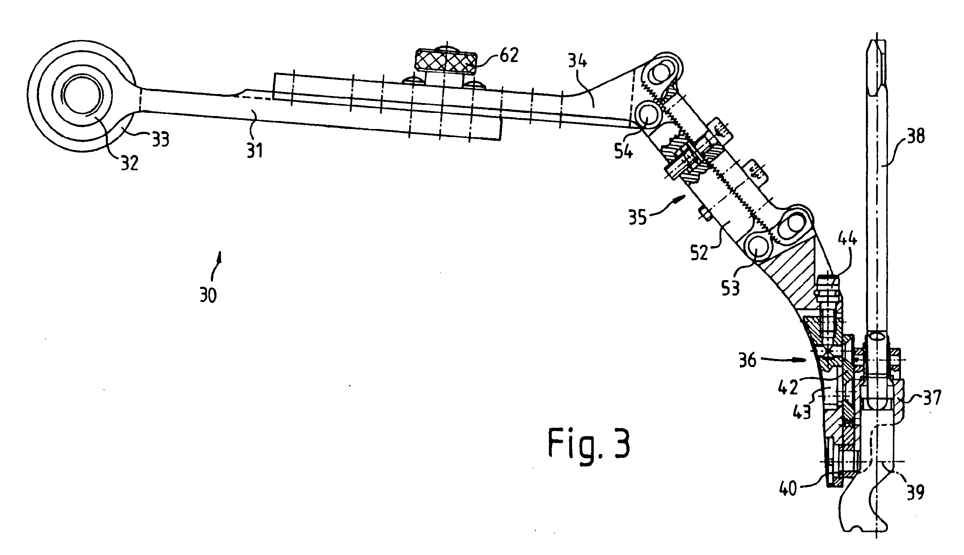

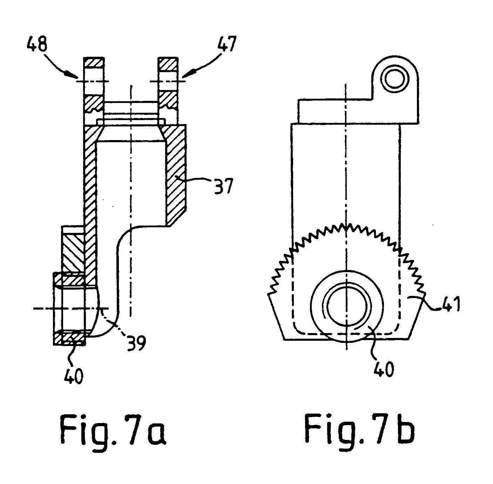

Der Spindelarm 30 gemäß Fig. 3 umfaßt eine Aufnahmeeinrichtung 31 mit Spindelbuchse 32 und Rändelrad 33 sowie ein daran verschiebbar und arretierbar angeordnetes Armteilstück 34 mit weiteren daran angelenkten Armteilstücken 35 und 36. Am Armteilstück 36 ist ein Aufnahmekörper 37 für einen Manipulationshebel 38 eines in einem Wirbelkörper eingesetzten Implantats (nicht dargestellt) vorgesehen. Der Aufnahmekörper 37 kann um eine Achse 39 senkrecht zur Längserstreckung des Armteilstücks 36 gedreht werden. Hierzu weist der Aufnahmekörper 37 einen Gelenkbolzen 40 auf, der im Armteilstück 36 aufgenommen wird (siehe Fig. 7a und b). Um den Gelenkbolzen 40 ist teilweise ein Zahnrad 41 ausgebildet, in welches zur Fixierung einer Drehstellung ein passend dazu ausgeformter Arretierungsstempel 42, der an einem Schieber 43 befestigt ist, eingreift. Der Schieber 43 mit Arretierungsstempel 42 kann mit Hilfe einer Spindel 44 fixiert werden.

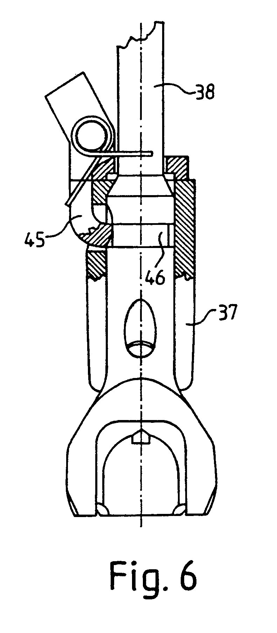

Die Befestigung eines Manipulationshebels 38 am Aufnahmekörper 37 ist in Figur 6 verdeutlicht. Dazu greift ein am Aufnahmekörper 37 federnd gelagerter Haken 45 in eine passend dazu ausgeformte umlaufende Nut 46 am Manipulationshebel 38 ein.

Lagerösen 47, 48 am Aufnahmekörper 37 für den Haken 45 sind insbesondere in Figur 7a zu sehen.

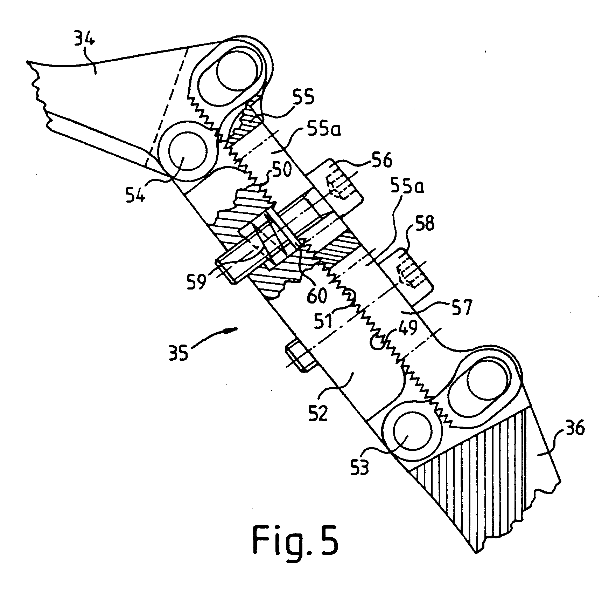

Die gelenkige Verbindung zwischen den Armteilstücken 34 und 36 über ein weiteres Armteilstück 35 erfolgt über gegenseitig blockierbare Zahnflächen 49, 50, 51 (siehe Fig. 5).

Die Zahnfläche 51 ist an einem Verbindungsstück 52 ausgebildet, das über die Gelenke 53 und 54 die Armteilstücke 34 und 36 verbindet. Am Armteilstück 34 ist jedoch zusätzlich ein Arretierungsstück 55 angelenkt, das die Zahnfläche 50 trägt. Über eine Schraube 56 können die Zahnflächen 51 und 50 gegeneinander verspannt werden, so dass der Winkel zwischen dem Armteilstück 34 und dem Verbindungsstück 52 des Armteilstücks 35 fixiert ist. In gleicher Weise ist am Armteilstück 36 ein Arretierungsstück 57 angeordnet, das die Zahnfläche 49 trägt und über ein Schraube 58 mit der Zahnfläche 51 des Verbindungsstücks 52 verspannt werden kann. Auf diese Weise wird der Winkel zwischen dem Verbindungsstück 52 des Armteilstücks 35 und dem Armteilstück 36 festgelegt. Um eine Verschiebung der Arretierungsstücke 55, 57 gegenüber dem Verbindungsstück 52 zu ermöglichen, sind in den Arretierungsstücken 55, 57 Langlöcher 55a ausgebildet, die von den Schrauben 56, 58 durchdrungen werden.

Damit sich die Zahnflächen 49, 50, 51 bei gelockerten Schrauben 56, 58 leicht voneinander lösen, ist im Verbindungsstück 52 jeweils eine Druckfeder 59 mit Druckscheibe 60 um die Schrauben 56, 58 angeordnet, die das jeweilige Arretierungsstück 55, 57 anhebt.

Im Weiteren weist das Armteilstück 34 eine Nut auf, die auf eine dazu passende Führungsschiene 61, welche an der Aufnahmeeinrichtung 31 ausgebildet ist, bei gelöster Rändelmutter 62 (siehe Fig. 3) aufgesteckt werden kann. Die Fixierung des Armteilstücks 34 an der Aufnahmeeinrichtung 31 erfolgt durch Anziehen der Rändelmutter 62.

Zur Verbesserung der Führung zwischen der Aufnahmeeinrichtung 31 und dem Armteilstück 34 sind in der Aufnahmeeinrichtung 31 Führungsbolzen 63 eingebracht, die in entsprechenden Langlöchern im Armteilstück 34 verschiebbar sind. Auf diese Weise wird auch eine Teleskopierbarkeit des Armes ermöglicht.

Die Achsen der Spindelbuchse 32 und der Gelenke 53 sowie 54 verlaufen parallel. Auf diese Weise benötigt jeder Arm unabhängig von der Winkelstellung seiner Armteilstücke bzw. der Aufnahmeeinrichtung in Holmlängsrichtung betrachtet immer den selben Platz, wodurch eine Behinderung von mehreren Armen, die dicht beieinander liegen vermieden wird.

### Bezugszeichenliste:

| | |
|---|---|
| 1 | Repositionsvorrichtung |
| 2 | Längsholm |
| 3 | Arm |
| 4 | Arm |
| 4a | Arm |
| 5 | Aufnahmeinrichtung |
| 6 | Rändelmutter |
| 7 | Armteilstück |
| 8 | Längsführung |
| 9 | Feststellschraube |
| 10 | Armteilstück |
| 11 | Armteilstück |
| 12 | Aufnahmekörper |
| 13 | Manipulationshebel |
| 14 | Gelenk |
| 15 | Gelenk |
| 16 | Achse |
| 17 | Rastfläche |
| 18 | Rastfläche |
| 19 | Spindel |
| 20 | Taste |
| 30 | Spindelarm |
| 31 | Aufnahmeeinrichtung |
| 32 | Spindelbuchse |
| 33 | Rändelrad |
| 34 | Armteilstück |
| 35 | Armteilstück |
| 36 | Armteilstück |
| 37 | Aufnahmekörper |
| 38 | Manipulationshebel |
| 39 | Achse |
| 40 | Gelenkbolzen |
| 41 | Zahnrad |
| 42 | Arretierungsstempel |
| 43 | Schieber |
| 44 | Spindel |
| 45 | Haken |
| 46 | Nut |
| 47 | Lageröse |
| 48 | Lageröse |
| 49 | Zahnfläche |
| 50 | Zahnfläche |
| 51 | Zahnfläche |
| 52 | Verbindungsstück |
| 53 | Gelenk |
| 54 | Gelenk |
| 55 | Arretierungsstück |
| 55a | Langloch |
| 56 | Schraube |
| 57 | Arretierungsstück |
| 58 | Schraube |
| 59 | Druckfeder |
| 60 | Druckscheibe |
| 61 | Führungsschiene |
| 62 | Rändelmutter |
| 63 | Führungsbolzen |

## Patentansprüche

1. Repositionsvorrichtung für die Wirbelsäule mit einem Längsholm, an welchem Arme angeordnet sind, wobei sich jeder Arm mit jeweils einem Implantat verbinden lässt, das in einem Wirbelkörper eingesetzt ist, wobei am Längsholm (2) mehr als zwei Arme (3, 4, 4a) derart angeordnet sind, dass die Arme (3,4,4a) Kraft in Holmlängsrichtung quer zur Armlängsrichtung aufnehmen können, welche eine Auslenkung der Arme in Richtung Längsholm verursachen würden, wobei wenigstens zwei Arme (3, 4, 4a) in Längsrichtung des Längsholms (2) am Längsholm verschiebbar sind, und wobei die Repositionseinrichtung eine Aufnahmevorrichtung (5) zur Anbringung am Längsholm (2) aufweist, an welche sich ein Armteilstück (7) anschließt und welche bei Abnahme des Armes (3, 4, 4a) am Längsholm (2) verbleibt,
**dadurch gekennzeichnet,**
**dass** an der Aufnahmevorrichtung (5) und an dem sich anschließenden Armteilstück (7) eine jeweils seitlich angeordnete Längsführung (8) so ausgebildet ist, dass die Aufnahmevorrichtung (5) und das Armteilstück (7) sich ineinander schieben lassen,
**dass** am Armteilstück (7) eine Befestigungsschraube (9) zur gegenseitigen Fixierung von Aufnahmevorrichtung (5) und Armteilstück (7) vorgesehen ist, und dass die Aufnahmevorrichtung (5) eine Rändelmutter (6) zur Verschiebung des Armes (3, 4, 4a) in Längsrichtung des Längsholmes (2) aufweist.

2. Repositionsvorrichtung nach Anspruch 1, **dadurch gekennzeichnet, dass** am Längsholm (2) mehr als zwei Arme (3, 4, 4a) derart angeordnet sind, dass sie Kräfte aufnehmen können, welche eine Verdrehung der Arme (3, 4, 4a) um die Längsachse des Holms (2) bewirken würden.

3. Repositionsvorrichtung nach einem der vorhergehenden Ansprüche, **dadurch gekennzeichnet, dass** wenigstens ein Arm (3, 4, 4a) bezüglich einer Verdrehung um die Längsachse des Längsholms (2) blockierbar ist.

4. Repositionsvorrichtung nach einem der vorhergehenden Ansprüche, **dadurch gekennzeichnet, dass** wenigstens ein Arm (3, 4, 4a) um die Längsachse verdrehsicher am Längsholm (2) angeordnet ist.

5. Repositionsvorrichtung nach einem der vorhergehenden Ansprüche, **dadurch gekennzeichnet, dass** wenigstens ein Arm (3, 4, 4a) ganz oder teilweise abnehmbar ist.

6. Repositionsvorrichtung nach Anspruch 5, **dadurch gekennzeichnet, dass** die Aufnahmeeinrichtung (5) vom Längsholm (2) durchdrungen wird.

7. Repositionsvorrichtung nach einem der vorhergehenden Ansprüche, **dadurch gekennzeichnet, dass** wenigstens ein Arm ein oder mehrere Gelenke (14, 15) umfasst, wobei sich die Gelenke (14, 15) blockieren lassen.

8. Repositionsvorrichtung nach Anspruch 7, **dadurch gekennzeichnet, dass** die Gelenkachsen parallel zum Längsholm (2) verlaufen.

9. Repositionsvorrichtung nach einem der vorhergehenden Ansprüche, **dadurch gekennzeichnet, dass** die Arme (3, 4, 4a) dergestalt sind, dass sie sich bezüglich des Längsholms (2) dreifach abwinkeln und anschließend blockieren lassen.

10. Repositionsvorrichtung nach einem der vorhergehenden Ansprüche, **dadurch gekennzeichnet, dass** die Arme (3, 4, 4a) verlängerbar, insbesondere teleskopierbar sind.

## Claims

1. A repositioning device for the spinal column comprising a longitudinal bar on which arms are arranged, wherein each arm can be connected to a respective implant inserted into a vertebra, wherein more than two arms (3, 4, 4a) are arranged on the longitudinal bar (2) in such a way that the arms (3, 4, 4a) can carry force in the longitudinal direction of the bar transversely to the longitudinal direction of the arms, which would cause deflection of the arms in the direction of the longitudinal bar, wherein at least two arms (3, 4, 4a) are displaceable on the longitudinal bar in the longitudinal direction of the longitudinal bar (2) and wherein the repositioning device has a receiving device (5) for mounting to the longitudinal bar (2), to which an arm portion (7) is joined and which remains on the longitudinal bar (2) upon removal of the arm (3, 4, 4a),
**characterised in that** provided at the receiving device (5) and at the joined arm portion (7) is a respective laterally arranged longitudinal guide (8) such that the receiving device (5) and the arm portion (7) can be pushed into each other,
provided on the arm portion (7) is a fixing screw (9) for mutual fixing of the receiving device (5) and the arm portion (7), and
the receiving device (5) has a knurled nut (6) for displacement of the arm (3, 4, 4a) in the longitudinal direction of the longitudinal bar (2).

2. A repositioning device according to claim 1 **characterised in that** more than two arms (3, 4, 4a) are arranged on the longitudinal bar (2) in such a way that they can carry forces which would cause rotation of the arms (3, 4, 4a) about the longitudinal axis of the bar (2).

3. A repositioning device according to one of the preceding claims **characterised in that** at least one arm (3, 4, 4a) can be blocked in regard to rotation about the longitudinal axis of the longitudinal bar (2).

4. A repositioning device according to one of the preceding claims **characterised in that** at least one arm (3, 4, 4a) is arranged on the longitudinal bar (2) non-rotatably about the longitudinal axis.

5. A repositioning device according to one of the preceding claims **characterised in that** at least one arm (3, 4, 4a) is entirely or partially removable.

6. A repositioning device according to claim 5 **characterised in that** the receiving device (5) has the longitudinal bar (2) passing therethrough.

7. A repositioning device according to one of the preceding claims **characterised in that** at least one arm includes one or more joints (14, 15), wherein the joints (14, 15) can be blocked.

8. A repositioning device according to claim 7 **characterised in that** the joint axes extend parallel to the longitudinal bar (2).

9. A repositioning device according to one of the preceding claims **characterised in that** the arms (3, 4, 4a) are of such a configuration that they can be angled three times with respect to the longitudinal bar (2) and then blocked.

10. A repositioning device according to one of the preceding claims **characterised in that** the arms (3, 4, 4a) can be lengthened, in particular they are telescopic.

## Revendications

1. Dispositif de repositionnement pour la colonne vertébrale, comprenant une barre longitudinale sur laquelle sont disposés des bras, chaque bras pouvant être relié à un implant qui est placé dans un corps vertébral, plus de deux bras (3, 4, 4a) étant disposés sur la barre longitudinale (2), de manière à ce que les bras (3, 4, 4a) puissent supporter des forces dans le sens longitudinal de la barre, transversalement au sens longitudinal des bras, forces qui provoqueraient une déviation des bras en direction de la barre longitudinale, au moins deux bras (3, 4, 4a) pouvant être déplacés sur la barre longitudinale, dans le sens longitudinal de la barre longitudinale (2), et le dispositif de repositionnement présentant un moyen de réception (5), destiné à une fixation sur la barre longitudinale (2), auquel se raccorde une portion de bras (7) et qui reste sur la barre longitudinale (2) lorsque le bras (3, 4, 4a) est retiré,
**caractérisé en ce que**
sur le moyen de réception (5) et sur la portion de bras (7) qui s'y raccorde, il est prévu un guide longitudinal (8) disposé respectivement sur le côté, de manière à ce que le moyen de réception (5) et la portion de bras (7) puissent être glissés l'un dans l'autre,
sur la portion de bras (7), il est prévu une vis de fixation (9) destinée à la fixation réciproque du moyen de réception (5) et de la portion de bras (7), et
le moyen de réception (5) présente un écrou moleté (6) pour déplacer le bras (3, 4, 4a) dans le sens longitudinal de la barre longitudinale (2).

2. Dispositif de repositionnement selon la revendication 1, **caractérisé en ce que** plus de deux bras (3, 4, 4a) sont disposés sur la barre longitudinale (2) de manière telle qu'ils puissent supporter des forces qui provoqueraient une rotation des bras (3, 4, 4a) autour de l'axe longitudinal de la barre (2).

3. Dispositif de repositionnement selon l'une des revendications précédentes, **caractérisé en ce qu'**au moins un bras (3, 4, 4a) peut être bloqué par rapport à une rotation autour de l'axe longitudinal de la barre longitudinale (2).

4. Dispositif de repositionnement selon l'une des revendications précédentes, **caractérisé en ce qu'**au moins un bras (3, 4, 4a) est disposé sur la barre longitudinale (2), en étant bloqué en rotation autour de l'axe longitudinal.

5. Dispositif de repositionnement selon l'une des revendications précédentes, **caractérisé en ce qu'**au moins un bras (3, 4, 4a) peut être retiré en partie ou en totalité.

6. Dispositif de repositionnement selon la revendication 5, **caractérisé en ce que** le moyen de réception (5) est traversé par la barre longitudinale (2).

7. Dispositif de repositionnement selon l'une des revendications précédentes, **caractérisé en ce qu'**au moins un bras comprend une ou plusieurs articulations (14, 15), sachant que les articulations (14, 15) peuvent être bloquées.

8. Dispositif de repositionnement selon la revendication 7, **caractérisé en ce que** les axes d'articulation s'étendent parallèlement à la barre longitudinale (2).

9. Dispositif de repositionnement selon l'une des revendications précédentes, **caractérisé en ce que** les bras (3, 4, 4a) sont agencés de manière telle qu'ils puissent être pliés trois fois par rapport à la barre longitudinale (2) et ensuite être bloqués.

10. Dispositif de repositionnement selon l'une des revendications précédentes, **caractérisé en ce que** les bras (3, 4, 4a) peuvent être rallongés, notamment de façon télescopique.
